# EUROPEAN PATENT APPLICATION

(11) **EP 4 799 649 A1**
(43) Date of publication of application: **02.09.2026**
(21) Application number: 24881724.9
(22) Date of filing: 25.10.2024
(51) Int. Cl.: A61K 47/68, C07K 19/00, C07K 16/18, A61K 39/395, A61P 37/00

(54) **METHOD FOR TREATING AUTOIMMUNE ENCEPHALITIS WITH TACI-FC FUSION PROTEIN**

(30) Priority: 27.10.2023 CN 202311411986
(71) Applicant: RemeGen Co., Ltd., Yantai, Shandong 264006 (CN)
(72) Inventor: FANG, Jianmin, Yantai, Shandong 264006 (CN); WANG, Wenxiang, Yantai, Shandong 264006 (CN); WU, Jingping, Yantai, Shandong 264006 (CN)
(74) Representative: Cooley (UK) LLP
(86) International application number: PCT/CN2024/127247
(87) International publication number: WO 2025/087363

(57) **Abstract**

A method for treating or alleviating autoimmune encephalitis with an effective amount of a medicament targeting Blys and/or APRIL. Results show that the effective amount of the medicament targeting Blys and/or APRIL exhibits a good safety profile and a relatively good therapeutic effect in the process of treating or alleviating autoimmune encephalitis.

## Description

### Technical Field

The present invention relates to a TACI-Fc fusion protein medicament for treating or alleviating autoimmune encephalitis, a dosage regimen, a dosing interval, and an administration mode therefor.

### Background Art

Autoimmune encephalitis (AE) refers to encephalitis caused by erroneous immune responses in which self-antigens expressed in the central nervous system (CNS) are recognized by the immune system as foreign antigens, and the main symptoms thereof include psychiatric and behavioral abnormalities, cognitive impairment, recent memory impairment, epileptic seizures, speech disorders, movement disorders, involuntary movements, decreased level of consciousness and coma, autonomic nervous system dysfunction, and the like. According to different anti-neuronal antibodies and corresponding clinical syndromes, autoimmune encephalitis is generally clinically classified into the following three main types: ① anti-NMDAR encephalitis, which is characterized clinically by diffuse encephalitis and differs from classic limbic encephalitis; ② limbic encephalitis, which has psychiatric and behavioral abnormalities, epileptic seizures and recent memory impairment as main symptoms, with electroencephalograms (EEGs) and imaging consistent with limbic system involvement, and encephalitis associated with anti-glutamic acid decarboxylase (GAD) antibodies, anti-LGI1 antibodies, anti-GABABR antibodies and anti-AMPAR antibodies conforming to the characteristics of limbic encephalitis; ③ other AE syndromes, including Morvan syndrome, anti-GABAAR antibody-associated encephalitis, progressive encephalomyelitis with rigidity and myoclonus (PERM), anti-DPPX antibody-associated encephalitis, anti-IgLON5 antibody-associated encephalopathy, and the like, and these AE syndromes either involve both the central and peripheral nervous systems or manifest as characteristic clinical syndromes.

Since the first discovery of anti-N-methyl-D-aspartate receptor (NMDAR) encephalitis in 2007, multiple novel pathogenic antibodies have been identified (Reference 1: Dalmau J, Graus F. Antibody-Mediated Encephalitis. N Engl J Med 2018; 378(9): 840-51), wherein anti-NMDAR encephalitis accounts for the highest proportion, approximately 54% to 80% of AE cases, followed by anti-leucine-rich glioma-inactivated protein 1 (LGI1) antibody-associated encephalitis and anti-γ-amino butyric acid type B receptor (GABABR) antibody-associated encephalitis, and the like (see Table 1).

**Table 1. Anti-neuronal cell antibodies associated with autoimmune encephalitis**

| CLASSIFICATION | Corresponding antigen | Antigen location | Age of onset (years) | Predominant gender | Encephalitis syndrome | Tumor proportion | Tumor Type |
|---|---|---|---|---|---|---|---|
| | NMDAR | Neuronal cell membrane | 1 to 85 | Female High proportion | Anti-NMDAR encephalitis | Females aged 12 to 45 years 40% | Ovarian teratoma |
| | LGI1 | Neuronal cell membrane | 15 to 96 | Male >60% | Limbic encephalitis | 5% to 10% | Thymoma |
| | GABABR | Neuronal cell membrane | 20 to 80 | Male Common | Limbic encephalitis | 50% | Small cell lung cancer |
| | CASPR2 | Neuronal cell membrane | 25 to 70 | Male >70% | Morvan syndrome, Limbic encephalitis | <10% | Thymoma |
| | IgLON5 | Neuronal cell membrane | 40 to 80 | Similar ratio of males to females | Encephalopathy with sleep disorder | <10% | - |
| Anti-cell surface antigen antibodies | AMPAR | Neuronal cell membrane | 35 to 85 | Female >70% | Limbic encephalitis | 60% | Small cell lung cancer, thymoma |
| | DPPX | Neuronal cell membrane | 45 to 75 | Male >60% | Encephalitis often accompanied by diarrhea | <10% | B-cell lymphoma |
| | GABAAR | Neuronal cell membrane | 1 to 75 | Similar ratio of males to females | Encephalitis | 25% | Thymoma |
| | mGluR5 | Neuronal cell membrane | 20 to 30 | Similar ratio of males to females | Encephalitis | 60% | Hodgkin lymphoma |
| | Neurexin-3α | Neuronal cell membrane | 40 to 50 | Female Common | Encephalitis | - | - |
| | D2R | Neuronal cell membrane | 0.5 to 17 | Similar ratio of males to females | Basal ganglia encephalitis | 0 | - |
| | GlyR | Neuronal cell membrane | 40 to 60 | Similar ratio of males to females | PERM | 20% | Thymoma |
| Anti-intracellular synaptic antigen antibodies | GAD | Neuronal cytoplasm | 10 to 85 | Female >70% | Limbic encephalitis | <10% | Thymoma, small cell lung cancer |
| | Amphiphysin | Neuronal cytoplasm | 60 to 70 | Slightly higher proportion of females | Limbic encephalitis | >80% | Small cell lung cancer, breast cancer |
| Anti-intracellular antigen antibodies | AK5 | Neuronal cytoplasm | 60 to 70 | Male Common | Limbic encephalitis | <10% | - |
| | Hu (ANNA-1) | Neuronal nucleus | 60 to 70 | Slightly higher proportion of females | Limbic encephalitis | >80% | Small cell lung cancer, neuroblast oma |
| | CV2/CRMP5 | Oligodend rocyte cytoplasm | 40 to 70 | Similar ratio of males to females | Limbic encephalitis | >80% | Small cell lung cancer, thymoma |
| | Ma2 | Neuronal nucleolus | Males 30-40 Females 60-70 | Male >70% | Limbic encephalitis, diencephalitis Encephalitis | >80% | Seminoma , non-small cell lung cancer |
| | KLHL11 | Neuronal nucleus | 40 to 50 | 100% male | Rhombencepha litis | >80% | Seminoma |

Autoimmune encephalitis may occur in adolescents, children, and adults, and research data show that the incidence of autoimmune encephalitis is 0.8 per 100,000 person-years, and the prevalence is 13.7/100,000 (Reference 2: Abboud H, Probasco JC, Irani S, et al. Autoimmune encephalitis: proposed best practice recommendations for diagnosis and acute management[J]. J Neurol Neurosurg Psychiatry, 2021.). Because some autoimmune encephalitis cases are associated with specific autoantibodies against cell surface molecules and intracellular targets, and testing does not always involve detection of antibodies, the sensitivity and specificity of detection vary, and test results also depend on the type of test performed. In addition, since antibody test results are usually negative, clinicians typically need to make a diagnosis by combining clinical manifestations, neuroimaging, electroencephalogram (EEG), and cerebrospinal fluid (CSF) results, and in CSF studies of some patients, antibody-mediated syndromes may not be associated with any evidence of inflammation, and the results of electroencephalogram are often nonspecific (Reference 3: Escudero D, Guasp M, Arino H, et al. Antibody-associated CNS syndromes without signs of inflammation in the elderly. Neurology 2017;89:1471-1475.). Therefore, high-quality diagnostic and therapeutic studies are still lacking at present.

According to the report of the Chinese Expert Consensus on the Diagnosis and Treatment of Autoimmune Encephalitis (2022 edition), the treatment of autoimmune encephalitis comprises immunotherapy, symptomatic treatment of epileptic seizures and psychiatric symptoms, supportive treatment, rehabilitation treatment, and anti-tumor treatment such as tumor resection for patients with concurrent tumors. Wherein, the immunotherapy can be further subdivided into first-line immunotherapy, second-line immunotherapy, long-term (maintenance) immunotherapy, escalation immunotherapy, and add-on immunotherapy. Table 2 summarizes the treatment regimens for immunotherapy, symptomatic treatment for seizures and psychiatric symptoms, and paraneoplastic AE.

**Table 2. Treatment Regimens for Autoimmune Encephalitis**

| | | | |
|---|---|---|---|
| Immunotherapy | First-line immunotherapy | Glucocorticoids, intravenous immunoglobulin (IVIg), and plasma exchange | Widely used in AE patients, and all first-onset AE patients should receive first-line immunotherapy. |
| | Second-line immunotherapy | Rituximab and intravenous cyclophosphamide | For severe patients with poor response to first-line immunotherapy. |
| | Long-term (maintenance) immunotherapy | Mycophenolate mofetil, azathioprine, and repeated rituximab | May be considered for patients whose condition shows no significant improvement after intensive first-line immunotherapy or second-line immunotherapy. |
| | Escalation immunotherapy | Intravenous tocilizumab | For refractory severe AE patients. |
| | Add-on immunotherapy | Intrathecal methotrexate, bortezomib, and low-dose interleukin-2 | For refractory severe AE patients, may be considered after screening if the condition shows no significant improvement after 1-2 months of second-line immunotherapy. |
| Symptomatic treatment for seizures | Benzodiazepines, sodium valproate, levetiracetam, lamotrigine, topiramate, carbamazepine, lacosamide, etc. | | |
| Symptomatic treatment for psychiatric symptoms | Olanzapine, clonazepam, sodium valproate, haloperidol, and quetiapine | | |
| Treatment of paraneoplastic AE | Medicaments acting on all lymphocytes (such as cyclophosphamide, mycophenolate mofetil, azathioprine) are generally selected, or medicaments acting primarily on T cells (such as tacrolimus, cyclosporine A, etc.) may be selected. | | |

As can be seen from Table 2, common first-line immunotherapies include glucocorticoids, intravenous immunoglobulin, and plasma exchange.
① Glucocorticoids (GC): Under normal circumstances, glucocorticoids are the first choice clinically for treating autoimmune encephalitis, and the principle is that glucocorticoids bind to specific receptors to induce T lymphocyte apoptosis and inhibit Th1 cell differentiation. When first-line medicaments are used in combination therapy, the most common combinations are glucocorticoids combined with intravenous immunoglobulin (IVIg) or glucocorticoids combined with plasma exchange. Although glucocorticoids have a therapeutic effect on AE, there is evidence that it is difficult to differentiate AE from infectious encephalitis in the acute phase, which often delays the use of glucocorticoids. In addition, the effect of glucocorticoids on reducing the number of circulating B cells is much less than that on reducing the number of T cells, and the impact on serum antibody titers is also relatively limited. In addition, long-term or high-dose glucocorticoids may induce or exacerbate psychiatric symptoms associated with adverse events, such as neurodegenerative diseases, depression, insomnia, agitation, and psychosis, and other complications, and the safety issues of immunotherapeutic medicaments need to be further explored.
② Immunosuppressants: For patients who are insensitive to first-line treatment, immunosuppressants are usually used for treatment, for example, cyclophosphamide, an immunosuppressant that can inhibit T cell and B cell proliferation, is generally administered by intravenous injection at a dose of generally 750 mg/m2 body surface area and dissolved in 100 mL of normal saline.
(3) Biologic products: Although various biologic products have been applied in clinical studies of autoimmune encephalitis (see Table 3), no drugs have been approved for marketing for this indication. In China, the use of biologic products such as rituximab and tocilizumab for autoimmune encephalitis is off-label, and special attention needs to be paid to possible infections when administering the drugs, and the patient's right to autonomous decision-making needs to be respected, and full informed consent and corresponding procedures need to be performed. Among them, rituximab is a chimeric monoclonal antibody against the CD20 antigen on the surface of B cells, and can kill B lymphocytes having CD20 by binding to CD20 and producing complement-dependent and antibody-dependent cytotoxic effects, and it also has an effect on the number and function of T cells. The recommended dose is 375 mg/m² of body surface area by intravenous infusion, once a week, and one course of treatment is 3-4 weeks, but special attention should be paid to complications and adverse reactions that may be induced by the use of rituximab. Other biological targeted therapies with good application prospects include satralizumab, rozanolixizumab and daratumumab, among which satralizumab is progressing the fastest and is currently in the Phase III clinical study stage (https://clinicaltrials.gov/study/NCT05503264?term=NCT05503264&rank=1), followed by rozanolixizumab, which is in the Phase II clinical study stage (https://www.clinicaltrials.gov/study/NCT02220153?term=NCT02220153&rank=1).

**Table 3. Biologic products for the treatment of autoimmune encephalitis**

| Generic Name | English Name | Clinical Progress | Target and Mechanism of Action |
|---|---|---|---|
| Satralizumab | Satralizumab | Phase III clinical trial | Anti-IL-6R monoclonal antibody |
| / | Rozanolixizumab | Phase II clinical trial | Anti-FcRn monoclonal antibody |
| Rituximab | Rituximab | / | Anti-CD20 monoclonal antibody |
| Tocilizumab | Tocilizumab | / | Anti-IL-6R monoclonal antibody |
| Daratumumab | Daratumumab | / | Anti-CD38 monoclonal antibody |

Since no medicament for the treatment of autoimmune encephalitis indications has currently been approved for marketing, the standards and guidelines for the clinical diagnosis of autoimmune encephalitis are derived from expert consensus, and there is also a lack of randomized controlled trials for the treatment of autoimmune encephalitis, and the level of evidence supporting immunotherapy medicaments is limited, and safety issues deserve in-depth research. In addition, biologic products such as rituximab may produce severe side effects such as infection, and therefore, there remains a real unmet clinical need for the treatment of autoimmune encephalitis, especially for patients who respond poorly to immunosuppressive medicaments, are intolerant to other biologic products, and experience severe adverse reactions, and there is an urgent clinical need to provide a safe and effective medicament.

Povetacicept (ALPN-303) is an Fc fusion protein of an engineered TACI domain, and as a BAFF/APRIL dual antagonist, it is being explored for the treatment of indications such as lupus nephritis (Phase I/II), membranous nephropathy (Phase I/II), IgA nephropathy (Phase I/II), immune thrombocytopenia (Phase I/II), cold agglutinin disease (Phase I/II), warm antibody autoimmune hemolytic anemia (Phase I/II), systemic lupus erythematosus (Phase I), autoimmune cytopenias (Phase I), and myasthenia gravis (preclinical), and is currently at different research stages.

Currently, Alpine Immune Sciences has disclosed information about three povetacicept clinical trials, wherein RUBY 1 (NCT05034484) is a Phase I safety study in healthy volunteers, demonstrating that povetacicept exhibits acceptable safety and tolerability profiles, as well as expected pharmacodynamic (PD) effects on circulating immunoglobulins (Ig). RUBY 3 (NCT05732402) is an open-label Phase 1b/2a study of povetacicept for the treatment of autoimmune kidney diseases, and the preliminary study results showed that povetacicept 80 mg SC Q4W, after multiple dosing in patients with IgA nephropathy, demonstrated good tolerability and is expected to reduce urinary protein-to-creatinine ratio (UPCR) and Gd-IgA1. RUBY 4 (NCT05757570) is an ongoing open-label Phase 1b study of povetacicept for the treatment of autoimmune cytopenias. In addition, povetacicept is intended for the treatment of inflammatory diseases involving BAFF and/or APRIL, such as systemic lupus erythematosus, and a clinical trial is being conducted in China (registration number CTR20234038).

Atacicept is a recombinant fusion protein comprising the extracellular ligand-binding portion of the TACI receptor and the Fc portion of human IgG, and can bind to Blys and APRIL. The drug is being explored for the treatment of indications such as IgA nephropathy (Phase III), lupus nephritis (Phase III), systemic lupus erythematosus (Phase II/III), autoimmune diseases, and is currently at different clinical stages.

The Phase 2b ORIGIN trial (NCT04716231) of atacicept for the treatment of IgA nephropathy obtained positive results, with atacicept treatment achieving the primary endpoint and key secondary endpoints. In addition, the safety profiles of the atacicept and placebo treatment groups were comparable. A Phase III clinical trial (NCT04716231) of atacicept for the treatment of IgA nephropathy is recruiting, with the treatment group receiving atacicept at a dose of 150 mg per administration, subcutaneously, once a week. In addition, a Phase IIb/III clinical trial (registration number: CTR20242150) evaluating the efficacy and safety of atacicept in subjects with IgA nephropathy (IgAN) is currently being conducted in China. Results from a Phase III clinical trial (NCT00624338) of atacicept for the efficacy and safety in prevention of moderate to severe systemic lupus erythematosus SLE showed that, compared with the placebo group, only the 150 mg dose atacicept group demonstrated efficacy (43% and 60%; OR 0.49 (0.26, 0.92), p=0.027), but due to two fatalities resulting from pneumonia complicated by pulmonary hemorrhage, the trial was terminated early for the atacicept 150 mg group. Three completed Phase II clinical trials of atacicept for the treatment of rheumatoid arthritis, NCT00595413, NCT00430495, and NCT00664521, were all terminated because the primary endpoints were not met.

Telitacicept (RC18) is a first-in-class recombinant TACI-Fc fusion protein for B cellrelated autoimmune diseases, which is capable of targeting and neutralizing two key cell signaling molecules in the B cell pathway, BLyS and APRIL. It is an antibody-like fusion protein composed of truncated TACI and an immunoglobulin Fc region optimized to exhibit reduced antibody-dependent cellular cytotoxicity (ADCC) and complement-dependent cytotoxicity (CDC) activities for the treatment of human autoimmune diseases, and has excellent biological activity and safety. It has been approved for marketing in China for the treatment of systemic lupus erythematosus, and its indication in combination with methotrexate for adult patients with moderate to severe active rheumatoid arthritis (RA) who have an inadequate response to methotrexate has also been approved for marketing.

### Summary of the Invention

The present invention has surprisingly discovered, during the course of treating real-world cases cases, that TACI-Fc fusion protein produced significant therapeutic effects when treating or alleviating autoimmune encephalitis in patients.

Therefore, the present invention provides a method for treating or alleviating autoimmune encephalitis, said method comprising administering a therapeutically effective amount of a medicament targeting Blys and/or APRIL to a patient having autoimmune encephalitis.

The present invention further provides the use of a medicament targeting Blys and/or APRIL in the preparation of a medicament for treating or alleviating autoimmune encephalitis in patients.

The present invention further provides the use of a TACI-Fc fusion protein in the preparation of a medicament for treating or alleviating autoimmune encephalitis in patients.

The present invention also provides the use of telitacicept in the preparation of a medicament for treating or alleviating autoimmune encephalitis in a patient.

Further, the above-mentioned medicament targeting Blys and/or APRIL is a TACI-Fc fusion protein.

Further, the TACI-Fc fusion protein according to any one of the above comprises:
(i) a TACI extracellular region or a fragment thereof that binds Blys and/or APRIL; and
(ii) a human immunoglobulin constant region fragment.

Further, the TACI extracellular region or a fragment thereof that binds Blys and/or APRIL comprises the amino acid sequence set forth in SEQ ID NO: 1 or SEQ ID NO:2 or SEQ ID NO:3.
SEQ ID NO:1 
SRVDQEERFP QGLWTGVAMR SCPEEQYWDP LLGTCMSCKT ICNHQSQRTC AAFCRSLSCR 60
KEQGKFYDHL LRDCISCASI CGQHPKQCAY FCENKLRSPV NLPPEL 106
SEQ ID NO:2 
AMRSCPEEQY WDPLLGTCMS CKTICNHQSQ RTCAAFCRSL SCRKEQGKFY DHLLRDCISC 60
ASICGQHPKQ CAYFCENKLR S 81
SEQ ID NO:3
   SLSCREKEQGE YYDHLLRDCI SCASICGQHP KQCADFCENK LRS 43

Further, the amino acid sequence of the TACI extracellular region or a fragment thereof that binds Blys and/or APRIL is set forth in SEQ ID NO:1.

Further, the human immunoglobulin is IgG1.

Further, the human immunoglobulin constant region fragment comprises the amino acid sequence of SEQ ID NO:4.

Further, the human immunoglobulin constant region fragment comprises an amino acid sequence having at least 90%, at least 91%, at least 92%, at least 93%, at least 94%, at least 95%, at least 96%, at least 97%, at least 98%, or at least 99% identity to SEQ ID NO:4.

SEQ ID NO:4
DKPHTCPLCP APELLGGPSV FLFPPKPKDT LMISRTPEVT CVVVDVSHED PEVKFNWYVD 60
GVEVHNAKTK PREEQYNSTY RVVSVLTVLH QDWLNGKEYK CKVSNKALPA PIEKTISKAK 120
GQPREPQVYT LPPSRDELTK NQVSLTCLVK GFYPSDIAVE WESNGQPENN YKATPPVLDS 180
DGSFFLYSKL TVDKSRWQQG NVFSCSVMHE ALHNHYTQKS LSLSPGK 227

Further, the amino acid sequence of the human immunoglobulin constant region fragment is set forth in SEQ ID NO:4.

Further, the human immunoglobulin constant region fragment comprises a modification of an amino acid on one or more positions corresponding to positions 3, 8, 14, 15, 17, 110, 111 or 173 of SEQ ID NO:4.

Further, the human immunoglobulin constant region fragment comprises amino acid modifications at 1, 2, 3, 4, 5, 6, 7, 8, or more positions as compared to SEQ ID NO:4.

Further, the modification is a substitution, deletion, or insertion of amino acids.

Further, the substitution comprises one or more of P3T, L8P, L14A, L15E, G17A, A110S, P111S, and A173T.

Further, the insertion is the insertion of 1, 2, 3, 4, 5, 6, 7, 8 or more amino acids at the N-terminus of the human immunoglobulin constant region fragment.

Still further, the insertion is the insertion of 5 amino acids at the N-terminus of the human immunoglobulin constant region fragment.

Still further, the insertion is the insertion of the 5 amino acids EPKSS at the N-terminus of the human immunoglobulin constant region fragment.

Further, the human immunoglobulin constant region fragment comprises the amino acid sequence of SEQ ID NO:5.

SEQ ID NO:5

DKTHTCPPCP APEAEGAPSV FLFPPKPKDT LMISRTPEVT CVVVDVSHED PEVKFNWYVD 60

GVEVHNAKTK PREEQYNSTY RVVSVLTVLH QDWLNGKEYK CKVSNKALPS SIEKTISKAK 120

GQPREPQVYT LPPSRDELTK NQVSLTCLVK GFYPSDIAVE WESNGQPENN YKTTPPVLDS 180

DGSFFLYSKL TVDKSRWQQG NVFSCSVMHE ALHNHYTQKS LSLSPGK 227

Further, the TACI-Fc fusion protein comprises the amino acid sequence set forth in SEQ ID NO:6.

SEQ ID NO:6

EPKSSDKTHT CPPCPAPEAE GAPSVFLFPP KPKDTLMISR TPEVTCVVVD VSHEDPEVKF 60

NWYVDGVEVH NAKTKPREEQ YNSTYRVVSV LTVLHQDWLN GKEYKCKVSN KALPAPIEKT 120

ISKAKGQPRE PQVYTLPPSR DELTKNQVSL TCLVKGFYPS DIAVEWESNG QPENNYKTTP 180

PVLDSDGSFF LYSKLTVDKS RWQQGNVFSC SVMHEALHNH YTQKSLSLSP G 231

Further, the TACI-Fc fusion protein has an amino acid sequence with at least 75%, at least 80%, at least 85%, at least 90%, at least 95% or at least 99% identity to SEQ ID NO:7.

SEQ ID NO:7
SRVDQEERFP QGLWTGVAMR SCPEEQYWDP LLGTCMSCKT ICNHQSQRTC AAFCRSLSCR 60
KEQGKFYDHL LRDCISCASI CGQHPKQCAY FCENKLRSPV NLPPELDKTH TCPPCPAPEA 120
EGAPSVFLFP PKPKDTLMIS RTPEVTCVVV DVSHEDPEVK FNWYVDGVEV HNAKTKPREE 180
QYNSTYRVVS VLTVLHQDWL NGKEYKCKVS NKALPSSIEK TISKAKGQPR EPQVYTLPPS 240
RDELTKNQVS LTCLVKGFYP SDIAVEWESN GQPENNYKTT PPVLDSDGSF FLYSKLTVDK 300
SRWQQGNVFS CSVMHEALHN HYTQKSLSLS PGK 333

Further, the amino acid sequence of the TACI-Fc fusion protein is set forth in SEQ ID NO:7.

Further, the TACI-Fc fusion protein has the amino acid sequence set forth in SEQ ID NO:8.

AMRSCPEEQY WDPLLGTCMS CKTICNHQSQ RTCAAFCRSL SCRKEQGKFY DHLLRDCISC 60
ASICGQHPKQ CAYFCENKLR SEPKSSDKTH TCPPCPAPEA EGAPSVFLFP PKPKDTLMIS 120
RTPEVTCVVV DVSHEDPEVK FNWYVDGVEV HNAKTKPREE QYNSTYRVVS VLTVLHQDWL 180
NGKEYKCKVS NKALPSSIEK TISKAKGQPR EPQVYTLPPS RDELTKNQVS LTCLVKGFYP 240
SDIAVEWESN GQPENNYKTT PPVLDSDGSF FLYSKLTVDK SRWQQGNVFS CSVMHEALHN 300
HYTQKSLSLS PGK 313

Further, the TACI-Fc fusion protein has the amino acid sequence set forth in SEQ ID NO:9.

SEQ ID NO:9

SLSCRKEQGE YYDHLLRDCI SCASICGQHP KQCADFCENK LRSGSGGGGS EPKSSDKTHT 60

CPPCPAPEAE GAPSVFLFPP KPKDTLMISR TPEVTCVVVD VSHEDPEVKF NWYVDGVEVH 120

NAKTKPREEQ YNSTYRVVSV LTVLHQDWLN GKEYKCKVSN KALPAPIEKT ISKAKGQPRE 180

PQVYTLPPSR DELTKNQVSL TCLVKGFYPS DIAVEWESNG QPENNYKTTP PVLDSDGSFF 240

LYSKLTVDKS RWQQGNVFSC SVMHEALHNH YTQKSLSLSP G 281

Further, the medicament targeting Blys and/or APRIL is telitacicept, atacicept, or povetacicept.

Further, the TACI-Fc fusion protein is telitacicept.

Further, the autoimmune encephalitis includes, but is not limited to, one or more of anti-NMDAR encephalitis, Morvan syndrome, progressive encephalomyelitis with rigidity and myoclonus, anti-dipeptidyl-peptidase-like protein-6 (DPPX) antibody encephalitis, anti-dopamine 2 receptor antibody-associated basal ganglia encephalitis, anti-LGI1 antibody-associated encephalitis, anti-GABABR antibody-associated encephalitis, anti-CASPR2 antibody-associated encephalitis, anti-IgLON5 antibody-associated encephalopathy, anti-AMPAR antibody-associated encephalitis, anti-GABAAR antibody-associated encephalitis, anti-mGluR5 antibody-associated encephalitis, anti-neurexin-3α antibody-associated encephalitis, anti-GAD65 encephalitis, anti-AK5 antibody-associated encephalitis, anti-Hu antibody-associated encephalitis, anti-CV2 antibody-associated encephalitis, anti-Ma2 antibody-associated encephalitis, anti-Kelch-like protein 11 antibody-associated encephalitis, anti-Ri antibody-associated encephalitis, anti-Yo antibody-associated encephalitis, anti-VGKC encephalitis, anti-VGCC encephalitis, and immune checkpoint inhibitor-associated encephalitis.

In some preferred embodiments, the autoimmune encephalitis is anti-NMDAR encephalitis; in other preferred embodiments, the autoimmune encephalitis is Morvan syndrome; in other preferred embodiments, the autoimmune encephalitis is progressive encephalomyelitis with rigidity and myoclonus; in other preferred embodiments, the autoimmune encephalitis is anti-dipeptidyl-peptidase-like protein-6 (DPPX) antibody encephalitis; in other preferred embodiments, the autoimmune encephalitis is anti-dopamine 2 receptor antibody-associated basal ganglia encephalitis; in other preferred embodiments, the autoimmune encephalitis is anti-LGI1 antibody-associated encephalitis; in other preferred embodiments, the autoimmune encephalitis is anti-GABABR antibody-associated encephalitis; in other preferred embodiments, the autoimmune encephalitis is anti-CASPR2 antibody-associated encephalitis; in other preferred embodiments, the autoimmune encephalitis is anti-IgLON5 antibody-associated encephalopathy; in other preferred embodiments, the autoimmune encephalitis is anti-AMPAR antibody-associated encephalitis; in other preferred embodiments, the autoimmune encephalitis is anti-GABAAR antibody-associated encephalitis; in other preferred embodiments, the autoimmune encephalitis is anti-mGluR5 antibody-associated encephalitis; in other preferred embodiments, the autoimmune encephalitis is anti-neurexin-3α antibody-associated encephalitis; in other preferred embodiments, the autoimmune encephalitis is anti-GAD65 encephalitis; in other preferred embodiments, the autoimmune encephalitis is anti-AK5 antibody-associated encephalitis; in other preferred embodiments, the autoimmune encephalitis is anti-CV2 antibody-associated encephalitis; in other preferred embodiments, the autoimmune encephalitis is anti-Ma antibody-associated encephalitis; in other preferred embodiments, the autoimmune encephalitis is anti-Kelch-like protein 11 antibody-associated encephalitis; in other preferred embodiments, the autoimmune encephalitis is anti-Ri antibody-associated encephalitis; in other preferred embodiments, the autoimmune encephalitis is anti-Yo antibody-associated encephalitis; in other preferred embodiments, the autoimmune encephalitis is anti-VGKC antibody-associated encephalitis; in other preferred embodiments, the autoimmune encephalitis is anti-VGCC encephalitis; in other preferred embodiments, the autoimmune encephalitis is immune checkpoint inhibitor-associated encephalitis; in other preferred embodiments, the autoimmune encephalitis is one of the above autoimmune encephalitides in combination with one or more other autoimmune encephalitides. It is understood that, clinically, autoimmune encephalitis may be classified into specific subtypes (i.e., further determination as one or two or more combinations of the above autoimmune encephalitides), or may remain unclassified (uniformly referred to as autoimmune encephalitis); therefore, the specific disease subtype should not be construed as a limitation to the present invention.

Further, the patient is an adult patient or a pediatric patient. In some preferred embodiments, the patient is an adult patient. In some other preferred embodiments, the patient is a pediatric patient.

Further, the patient is an antibody-positive autoimmune encephalitis patient or an antibody-negative autoimmune encephalitis patient. In some preferred embodiments, the patient is an antibody-positive autoimmune encephalitis patient. In other preferred embodiments, the patient is an antibody-negative autoimmune encephalitis patient.

Further, the antibody-positive autoimmune encephalitis patient is a patient with autoimmune encephalitis positive for one or more antibodies.

Further, the autoimmune encephalitis patient is in a new-onset disease treatment stage or a relapsing disease treatment stage. In some preferred embodiments, the autoimmune encephalitis patient is in a new-onset disease treatment stage (i.e., the patient is diagnosed with autoimmune encephalitis for the first time). In other preferred embodiments, the autoimmune encephalitis patient is in a relapsing disease treatment stage. That is to say, the medicament targeting Blys and/or APRIL provided by the present invention (further preferably a TACI-Fc fusion protein, and still further preferably telitacicept) can be applied to both the new-onset disease treatment stage of patients and the relapsing disease treatment stage of patients.

Further, the autoimmune encephalitis patient concurrently suffers from one or more other autoimmune diseases. Wherein the autoimmune diseases (with reference to the disease list published by the American Autoimmune Related Diseases Association) include, but are not limited to: psoriasis, Hashimoto's thyroiditis, systemic lupus erythematosus, rheumatoid arthritis, type 1 diabetes mellitus, toxic diffuse goitre (Graves' disease), inflammatory bowel disease, coeliac disease, achalasia, Addison's disease, adult-onset Still's disease, agammaglobulinaemia, alopecia areata, amyloidosis, ankylosing spondylitis, anti-glomerular basement membrane nephritis, antiphospholipid syndrome, autoimmune angioedema, autoimmune autonomic dysfunction, autoimmune encephalomyelitis, autoimmune hepatitis, autoimmune inner ear disease, autoimmune myocarditis, autoimmune oophoritis, autoimmune orchitis, autoimmune pancreatitis, autoimmune retinopathy, autoimmune urticaria, acute motor and sensory axonal neuropathy, Balo's disease, Behçet's disease, benign mucous membrane pemphigoid, bullous pemphigoid, Castleman disease, coeliac disease, Chagas disease, chronic inflammatory demyelinating polyneuropathy, chronic recurrent multifocal osteomyelitis, Churg-Strauss syndrome (eosinophilic granulomatosis with polyangiitis), Cogan's syndrome, cold agglutinin disease, congenital heart block, Coxsackie virus myocarditis, CREST syndrome, Crohn's disease, dermatitis herpetiformis, dermatomyositis, neuromyelitis optica (Devic's disease), discoid lupus erythematosus, Dressler's syndrome, endometriosis, eosinophilic esophagitis, eosinophilic fasciitis, erythema nodosum, primary mixed cryoglobulinemia, Evans syndrome, fibromyalgia, fibrosing alveolitis, giant cell arteritis, giant cell myocarditis, glomerulonephritis, Goodpasture syndrome, granulomatosis with polyangiitis, Graves' disease, Guillain-Barré syndrome, Hashimoto's thyroiditis, hemolytic anemia, Henoch-Schonlein purpura, herpes gestationis (pemphigoid gestationis), hidradenitis suppurativa, hypogammaglobulinemia, IgA nephropathy, IgG4-related sclerosing disease, immune thrombocytopenic purpura, inclusion body myositis, interstitial cystitis, juvenile arthritis, juvenile myositis, Kawasaki disease, Lambert-Eaton syndrome, leukocytoclastic vasculitis, lichen planus, lichen sclerosus et atrophicus, ligneous conjunctivitis, linear IgA disease, chronic Lyme disease, Ménière's disease, microscopic polyangiitis, mixed connective tissue disease, Mooren's ulcer, Mucha-Habermann disease, multifocal motor neuropathy, multiple sclerosis, myasthenia gravis, myositis, narcolepsy, neonatal lupus, neutropenia, ocular cicatricial pemphigoid, optic neuritis, relapsing rheumatic disease, Behçet's disease, paraneoplastic cerebellar degeneration, paroxysmal nocturnal hemoglobinuria, Parry-Romberg syndrome, pars planitis, Parsonage-Turner syndrome, pemphigus, peripheral neuropathy, perivenous encephalomyelitis, pernicious anemia, POEMS syndrome, polyarteritis nodosa, autoimmune polyendocrine syndrome type I, type II, or type III, polymyalgia rheumatica, polymyositis, post-myocardial infarction syndrome, post-pericardiotomy syndrome, primary biliary cirrhosis, primary sclerosing cholangitis, autoimmune progesterone dermatitis, psoriatic arthritis, pure red cell aplasia, pyoderma gangrenosum, Raynaud's phenomenon, reactive arthritis, reflex sympathetic dystrophy, relapsing polychondritis, restless legs syndrome, retroperitoneal fibrosis, rheumatic fever, sarcoidosis, Schmidt syndrome, scleritis, scleroderma, Sjögren's syndrome, autoimmune orchitis and autoimmune infertility, stiff-person syndrome, subacute bacterial endocarditis, Susac syndrome, sympathetic ophthalmia, Takayasu arteritis, temporal arteritis, thyroid eye disease, Tolosa-Hunt syndrome, ulcerative colitis, undifferentiated connective tissue disease, uveitis, vasculitis, vitiligo, Vogt-Koyanagi-Harada disease, and antibody-mediated autoimmune hemolytic anemia.

Further, the autoimmune encephalitis patient concurrently suffers from one or more other tumor diseases. Wherein, the tumor disease is a solid tumor or a non-solid tumor, including but not limited to breast cancer, ovarian cancer, cervical cancer, uterine cancer, prostate cancer, renal cancer, urethral cancer, bladder cancer, liver cancer, gastric cancer, endometrial cancer, salivary gland cancer, esophageal cancer, lung cancer, colon cancer, rectal cancer, colorectal cancer, bone cancer, skin cancer, thyroid cancer, pancreatic cancer, melanoma, glioma, neuroblastoma, glioblastoma multiforme, sarcoma, lymphoma, teratoma, testicular germ cell tumor, mediastinal hamartoma, small cell lung cancer and leukemia, and other solid tumors or hematologic malignancies.

Further, the patient has not previously received an autoimmune encephalitis treatment regimen.

Further, the patient has previously received an autoimmune encephalitis treatment regimen.

Further, the autoimmune encephalitis treatment regimen includes but is not limited to one or more of a glucocorticoid treatment regimen, an immunosuppressant treatment regimen, a biologic product treatment regimen, and plasma exchange.

In some preferred embodiments, the patient has previously received an autoimmune encephalitis treatment regimen, wherein the autoimmune encephalitis treatment regimen is a glucocorticoid treatment regimen, an immunosuppressant treatment regimen, a biologic product treatment regimen, plasma exchange, a glucocorticoid + immunosuppressant combination regimen, a glucocorticoid + biologic product combination regimen, a glucocorticoid + plasma exchange combination regimen, an immunosuppressant + biologic product combination regimen, an immunosuppressant + plasma exchange combination regimen, a biologic product + plasma exchange combination regimen, a glucocorticoid + immunosuppressant + biologic product combination regimen, a glucocorticoid + immunosuppressant + plasma exchange combination regimen, an immunosuppressant + biologic product + plasma exchange combination regimen, or a glucocorticoid + immunosuppressant + biologic product + plasma exchange combination regimen.

Further, the method comprises administering to the patient having the autoimmune encephalitis a therapeutically effective amount of a medicament targeting Blys and/or APRIL in combination with one or more of a glucocorticoid, an immunosuppressant, biologic product, and plasma exchange.

In some preferred embodiments, the method comprises administering a therapeutically effective amount of a medicament targeting Blys and/or APRIL to the patient having the autoimmune encephalitis. In other preferred embodiments, the method comprises concomitantly administering a medicament targeting Blys and/or APRIL, a glucocorticoid and an immunosuppressant to the patient having the autoimmune encephalitis; or concomitantly administering a medicament targeting Blys and/or APRIL, a glucocorticoid and a biologic product; or concomitantly administering a medicament targeting Blys and/or APRIL, a glucocorticoid and plasma exchange; or concomitantly administering a medicament targeting Blys and/or APRIL, an immunosuppressant and a biologic product; or concomitantly administering a medicament targeting Blys and/or APRIL, an immunosuppressant and plasma exchange; or concomitantly administering a medicament targeting Blys and/or APRIL, a biologic product and plasma exchange; or concomitantly administering a medicament targeting Blys and/or APRIL, a glucocorticoid, an immunosuppressant and a biologic product; or concomitantly administering a medicament targeting Blys and/or APRIL, a glucocorticoid, an immunosuppressant and plasma exchange; or concomitantly administering a medicament targeting Blys and/or APRIL, an immunosuppressant, a biologic product and plasma exchange. Preferably, the concomitant administration is simultaneous application or staged application.

The glucocorticoid refers to a regulatory molecule that plays an important regulatory role in the development, growth, metabolism and immune function of the body, is the most important regulatory hormone of the stress response of the body, and is also the most widely used and effective anti-inflammatory agent clinically. Preferably, the glucocorticoid involved in the present invention includes but is not limited to: prednisone, prednisone, methylprednisolone, betamethasone, beclomethasone propionate, prednisolone, hydrocortisone, dexamethasone, and the like.

The "immunosuppressant" is a medicament that has an inhibitory effect on the immune response of the body, can inhibit the proliferation and function of cells related to immune responses (macrophages such as T cells and B cells), and is also referred to as conventional synthetic diseasemodifying anti-rheumatic drugs (cDMARDs). Preferably, the immunosuppressants in the present invention include, but are not limited to, medicaments that have an inhibitory effect on the immune response of the body, such as mycophenolate mofetil, azathioprine, methotrexate, leflunomide, cyclophosphamide, cyclosporine, tacrolimus, iguratimod, thalidomide, and the like.

Preferably, the biologic products of the present invention include, but are not limited to, one or more of rituximab, satralizumab, rozanolixizumab, tocilizumab, daratumumab, human immunoglobulin and gamma globulin.

Further, the single dose of the medicament targeting Blys and/or APRIL is from about 0.1 to 10 mg/kg, further comprising 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, 10 mg/kg.

Further, the single dose of the medicament targeting Blys and/or APRIL is 40-240 mg, further preferably 40 mg, 50 mg, 60 mg, 70 mg, 80 mg, 90 mg, 100 mg, 110 mg, 120 mg, 130 mg, 140 mg, 150 mg, 160 mg, 170 mg, 180 mg, 190 mg, 200 mg, 210 mg, 220 mg, 230 mg, 240 mg.

Further, the method for detecting the protein content of the above medicament is: ultraviolet-visible spectrophotometry, wherein the absorbance value of the telitacicept sample at this wavelength is measured based on the fact that proteins have maximum ultraviolet absorption at 280 nm. After correcting for the absorbance at 320 nm, the absorbance value at 280 nm is proportional to the protein concentration, and the protein concentration is calculated according to the Lambert-Beer law to determine the protein content. The formula for calculating the protein content is as follows: $Protein content \left(mg/ml\right) = \frac{A 280 \left(or A280 \left(corrected\right)\right)}{ε} ∗ Sample dilution factor$
wherein: ε is the extinction coefficient of telitacicept, and has a unit of (mg/ml)⁻¹•cm⁻¹;
A₂₈₀ is the average absorbance of the sample solution at 280 nm;
A₂₈₀ (corrected) is the average corrected absorbance of the sample solution at 280 nm.

Further, the medicament targeting Blys and/or APRIL is administered 1-5 times per month, that is, the dosing frequency of the TACI-Fc fusion protein is once, twice, three times, four times, or five times per month.

Further, the medicament targeting Blys and/or APRIL is administered 1-10 times per two-month interval, that is, the dosing frequency of the TACI-Fc fusion protein is once, twice, three times, four times, five times, six times, seven times, eight times, nine times, or ten times per two months.

Further, the medicament targeting Blys and/or APRIL is administered 1-10 times per three-month interval, that is, the dosing frequency of the TACI-Fc fusion protein is once, twice, three times, four times, five times, six times, seven times, eight times, nine times, ten times, eleven times, twelve times, thirteen times, fourteen times, or fifteen times per three months.

Further, the dosing frequency of the medicament targeting Blys and/or APRIL is once a week.

Further, the dosing frequency of the medicament targeting Blys and/or APRIL is as-needed dosing.

Further, the medicament targeting Blys and/or APRIL is administered continuously and/or intermittently.

Further, the medicament targeting Blys and/or APRIL is administered at regular intervals.

Further, the medicament targeting Blys and/or APRIL is administered at irregular intervals.

Further, the administration mode of the medicament targeting Blys and/or APRIL is subcutaneous, intramuscular or intravenous administration, and the administration site is preferably the thigh, the abdomen, or the upper arm. In some specific embodiments, the administration mode of the TACI-Fc fusion protein is subcutaneous injection, intramuscular injection or intravenous injection.

Further, the injection site of the medicament targeting Blys and/or APRIL is the same or different for each injection. In some specific embodiments, the injection site of the TACI-Fc fusion protein is the same for each injection; in other specific embodiments, the injection site of the TACI-Fc fusion protein is different for each injection.

The present invention further provides a method for treating a patient with autoimmune encephalitis who has previously received an autoimmune encephalitis treatment regimen, the method comprising (1) determining whether the patient has previously received an autoimmune encephalitis treatment regimen, and (2) if the patient has previously received an autoimmune encephalitis treatment regimen, administering to the patient having autoimmune encephalitis an effective amount of a medicament targeting Blys and/or APRIL, further being a TACI-Fc fusion protein.

### Detailed Description of Embodiments

Unless defined otherwise, all terms used herein have the same meanings as commonly understood by one of ordinary skill in the art. With regard to definitions and terminology in the art, professionals may specifically refer to Current Protocols in Molecular Biology (Ausubel).

The three-letter code and one-letter code for amino acids used in the present invention are as described in J. Biol. Chem., 243, p3558 (1968).

The term "TACI" in the present invention, i.e., transmembrane activator and CAML interactor, is one of the members of the tumor necrosis factor receptor superfamily. The term "BLys" in the present invention refers to B lymphocyte stimulator, which is a member of the TNF ligand superfamily that exists in two forms, membrane-bound and soluble, which is specifically expressed on the surface of bone marrow cells, and selectively stimulates B lymphocyte proliferation and immunoglobulin production. The term "APRIL" (a proliferation-inducing ligand) in the present invention is a tumor necrosis factor (TNF) analog that can stimulate the proliferation of naive B cells and T cells in the body, promote B cell accumulation, and increase spleen content. APRIL can specifically bind to TACI and BCMA, and after binding can prevent APRIL from binding to B cells and inhibit APRIL-stimulated naïve B cell proliferation response. Moreover, APRIL can compete with BLys for binding to receptors (BCMA, TACI).

The term "TACI-Fc fusion protein" involved in the present invention refers to transmembrane activator, calcium modulator and cyclophilin ligand interactor (TACI)-immunoglobulin fusion protein (i.e., TACI-Fc fusion protein), and the TACI-immunoglobulin fusion protein provided by the present invention comprises: (i) the TACI extracellular region or a fragment thereof that binds Blys and/or APRIL; and (ii) a human immunoglobulin constant region fragment. Examples of the TACI-Fc fusion protein include, but are not limited to, telitacicept (amino acid sequence: SEQ ID NO: 7), atacicept (amino acid sequence: SEQ ID NO: 8), povetacicept (amino acid sequence: SEQ ID NO: 9).

The term "TACI extracellular region or a fragment thereof that binds Blys and/or APRIL" in the present invention includes the extracellular domain of TACI in U.S. Patent Nos. 5,969,102, 6,316,222, and 6,500,428 and U.S. Patent Applications 09/569,245 and 09/627,206 (the contents of which are incorporated herein by reference), as well as specific fragments of the TACI extracellular domain capable of interacting with TACI ligands, and the amino acid fragment at positions 13-118 of the TACI extracellular domain disclosed in Chinese Patent Publication No. CN101323643A.

In the term "human immunoglobulin constant region fragment," the immunoglobulin portion is preferably IgG1, which may comprise a heavy chain constant region, such as a human heavy chain constant region. The preferred "human immunoglobulin constant region fragment" of the present invention is an amino acid fragment containing a partial hinge region domain, a CH2 domain, and a CH3 domain. In some more preferred embodiments, the amino acid sequence of the "human immunoglobulin constant region fragment" according to the present invention is set forth in SEQ ID NO:4, or comprises an amino acid sequence having at least 90%, at least 91%, at least 92%, at least 93%, at least 94%, at least 95%, at least 96%, at least 97%, at least 98%, or at least 99% identity to SEQ ID NO:4. In some more preferred embodiments, the amino acid sequence of the "human immunoglobulin constant region fragment" is set forth in SEQ ID NO:5.

The term "treatment" referred to in the present invention is associated with a given disease or condition, and includes but is not limited to: inhibiting the disease or condition, for example, arresting the development of the disease or condition; relieving the disease or condition, for example, causing regression of the disease or condition; or alleviating symptoms caused by the disease or condition, for example, alleviating, preventing or treating the symptoms of the disease or condition; or reducing the chance of disease relapse or preventing disease relapse.

The term "remission" in the present invention refers to the absence of clinical symptoms or signs related to autoimmune encephalitis at the beginning or end of treatment.

The term "amino acid" as used in the present invention is understood in the broadest sense as a general term for a class of organic compounds containing an amino group and a carboxyl group, and preferably, the amino acids involved in the present invention are the main units that constitute proteins in living organisms, including but not limited to: glycine, alanine, valine, leucine, isoleucine, methionine, proline, tryptophan, serine, tyrosine, cysteine, phenylalanine, asparagine, glutamine, threonine, aspartic acid, glutamic acid, lysine, arginine and histidine.

The three-letter code and one-letter code for amino acids used in the present invention are as described in J. Biol. Chem., 243, p3558 (1968). There are multiple numbering schemes for amino acid positions, such as the Kabat numbering system, the EU numbering system, sequential numbering, and the like, and in the present invention, the amino acid positions are numbered using "sequential numbering", "positions 3, 8, 14, 15, 17, 110, 111 or 173 of SEQ ID NO:4" as described in the present invention refers to the amino acid at position 3, the amino acid at position 8 of SEQ ID NO:4, and so on; and "P3T" as described in the present invention refers to mutating the amino acid sequence at position 3 of SEQ ID NO:4 from the original "P" to "T", and "L8P" refers to mutating the amino acid sequence at position 8 of SEQ ID NO:4 from the original "L" to "P", and so on.

As an alternative embodiment, the constant region of the immunoglobulin provided by the present invention may introduce alterations of one or more amino acids, such as substitution (i.e. mutation), addition (i.e. insertion) or deletion.

The term "telitacicept" (also referred to as "Tai'ai" or RC18, which are used interchangeably in the present invention) is a TACI-Fc fusion protein whose INN is telitacicept, the amino acid sequence of which is set forth in SEQ ID NO: 7, or see https://extranet.who.int/soinn/mod/page/view.php?id=137&inn_n=10932.

The term "atacicept" in the present invention is a TACI-Fc fusion protein whose INN is atacicept, the amino acid sequence of which is set forth in SEQ ID NO: 8, or see https://extranet.who.int/soinn/mod/page/view.php?id=137&inn_n=8669.

The term "povetacicept" in the present invention, i.e., ALPN-303, is a TACI-Fc fusion protein whose INN is povetacicept, the amino acid sequence of which is set forth in SEQ ID NO: 9, or see https://extranet.who.int/soinn/mod/page/view.php?id=137&inn_n=12264.

The TACI-Fc fusion protein of the present invention can be administered by any of a variety of routes, including but not limited to: oral, intravenous injection, intramuscular injection, intraarterial injection, intramedullary injection, intraperitoneal injection, intrathecal injection, intracerebrocardiac, transdermal, percutaneous, topical, subcutaneous, intranasal, enteral, sublingual, intravaginal or rectal routes and the like.

The term "autoimmune encephalitis" in the present invention refers to a disease caused by the immune system reacting against central nervous system antigens, characterized by acute or subacute onset of epilepsy, cognitive impairment, and psychiatric disorders as the main clinical features.

The term "anti-NMDAR encephalitis" in the present invention refers to anti-N-methyl-D-aspartate receptor encephalitis, which is characterized by the presence of IgG subtype anti-NMDAR autoantibodies in the cerebrospinal fluid. Most patients with anti-NMDAR encephalitis progress to multi-stage disease, from psychiatric abnormalities, epilepsy, language disorder to an unresponsive state.

The term "Morvan syndrome" (also known as Morvan syndrome) in the present invention refers to an autoimmune disease clinically characterized by hyperexcitability of the central nervous system, peripheral nervous system, and autonomic nervous system, anti-CASPR2 antibodies play an important pathogenic role in Morvan syndrome, CASPR2 is a protein expressed in the central nervous system and paranodal regions of myelinated nerves, and detection of anti-CASPR2 antibodies in serum and/or cerebrospinal fluid is of great significance for definitive diagnosis of this disease.

The term "progressive encephalomyelitis with rigidity and myoclonus" in the present invention refers to a life-threatening autoimmune disease characterized by rigidity, muscle pain and spasm, deep and superficial sensory disturbances and brainstem-spinal cord symptoms, autonomic function, dyspnea, sudden spontaneous, and stimulus-induced myoclonus.

The term "anti-dipeptidyl-peptidase-like protein-6 antibody encephalitis" in the present invention is also referred to as anti-DPPX (dipeptidyl-peptidase-like protein-6) antibody encephalitis, which is a chronic or subacute progressive autoimmune encephalitis mediated by DPPX antibodies in serum and/or cerebrospinal fluid, the clinical manifestations are complex and diverse, gastrointestinal dysfunction, cognitive-psychiatric disorders and central nervous system hyperexcitability are typical manifestations, lymphoma may occur concomitantly, immunotherapy is effective but relapse is prone to occur.

The term "anti-dopamine 2 receptor (D2R) antibody-associated basal ganglia encephalitis" in the present invention is a type of anti-neuronal surface antibody-associated autoimmune encephalitis, with lesions predominantly involving the basal ganglia region. The main manifestations are movement disorders and psychiatric disorders characterized by Parkinsonism, dystonia and chorea.

The term "anti-LGI1 antibody-associated encephalitis" in the present invention refers to leucine-rich-glioma-inactivated protein 1 (LGI1) antibody-associated encephalitis, which belongs to a type of limbic encephalitis, and LGI1 antibody is an autoantibody-mediated anti-cell membrane antigen antibody. The specific symptom is faciobrachial dystonic seizure, which manifests as brief and frequent involuntary movements of the unilateral face, arm and lower limb.

The term "anti-GABABR antibody-associated encephalitis" in the present invention refers to anti-γ-aminobutyric acid B type receptor (GABABR) antibody-associated encephalitis, GABABR antibody belongs to cell surface antibodies, is often accompanied by marked seizure or status epilepticus, and some patients have concurrent tumors, mainly small cell lung cancer.

The anti-CASPR2 antibody in the term "anti-CASPR2 antibody-associated encephalitis" in the present invention is associated with peripheral nerve hyperexcitability (such as myokymia, fasciculation, muscle spasm) and encephalitis. Other symptoms in patients with this disease include autonomic nervous system dysfunction and agrypnia excitata. Nearly one-third of patients present with Morvan syndrome, namely distal upper limb movement disorders, peripheral nerve hyperexcitability, autonomic nervous system dysfunction, pain and encephalitis.

In the term "anti-IgLON5 antibody-associated encephalopathy" in the present invention, IgLON5 antibody-positive patients present with unique non-rapid eye movement (NREM) and rapid eye movement (REM) sleep abnormalities, accompanied by obstructive sleep apnea, stridor, episodic central hypoventilation, dementia, gait instability, chorea, dysarthria, dysphagia, autonomic disorder and supranuclear gaze palsy.

The term "anti-AMPAR antibody-associated encephalitis" in the present invention refers to anti-α-amino-3-hydroxy-5-methyl-4-isoxazolepropionic acid receptor antibody-associated encephalitis, wherein AMPAR is a tetramer composed of four subunits GluR1-4 (GluRA-D), and the main clinical manifestations are acute/subacute, with relatively mild effects on cognitive ability, attention, or language function.

The term "anti-GABAAR antibody-associated encephalitis" in the present invention has as its main symptom refractory, uncontrollable epilepsy, which often progresses to refractory status epilepticus, and is often accompanied by cognitive dysfunction and behavioral changes. decreased consciousness and movement disorders.

The term "anti-mGluR5 antibody-associated encephalitis" in the present invention refers to anti-metabotropic glutamate receptor 5 antibody-associated encephalitis. Metabotropic glutamate receptor 5 belongs to the Group I subclass of the metabotropic glutamate receptor family, is widely expressed in the postsynaptic membrane of neurons in brain regions responsible for memory and learning (such as cerebral cortex, hippocampus, basal ganglia, etc.), and plays a key role in regulating synaptic transmission and plasticity. Anti-mGluR5-associated encephalitis is extremely rare, most cases are associated with Hodgkin lymphoma, neurological symptoms usually appear earlier than tumor diagnosis, but it can also be associated with other tumors such as small cell lung cancer or be non-paraneoplastic.

The neurexin in the term "anti-neurexin-3α antibody-associated encephalitis" in the present invention is a family of synaptic cell adhesion molecules, plays a key role in synaptic development and function, is encoded by three genes NRXN1, NRXN2 and NRXN3, each gene can encode and produce two fragment products of different lengths, wherein the longer fragment is α. Neurexin-3α can specifically bind to leucine-rich repeat transmembrane neuronal protein 2, mediating calcium ion-induced neurotransmitter release. Neurexin-3α antibodies can lead to a reduction of neurexin-3α on the nerve surface, but do not lead to a reduction in the number of synapses, thereby causing synaptic excitatory and inhibitory dysfunction, and patients are accompanied by cognitive decline, psychiatric and behavioral abnormalities, refractory epilepsy, autonomic nervous system dysfunction (tachycardia, rapid respiratory rate), and symptoms such as involuntary movements around the mouth and on the ipsilateral limbs.

The term "anti-GAD65 encephalitis" in the present invention refers to "anti-glutamic acid decarboxylase (GAD) 65 encephalitis", and GAD65 catalyzes the conversion of glutamic acid into the neurotransmitter GABA. Anti-GAD65 antibodies are associated with other extra-neurological autoimmune diseases, such as type I diabetes. The main neurological diseases associated with anti-GAD65 antibodies include stiff-person syndrome, cerebellar ataxia, epilepsy, and limbic encephalitis.

The term "anti-AK5 antibody-associated encephalitis" in the present invention refers to limbic encephalitis with anti-adenylate kinase 5 antibodies, mainly affecting middle-aged and elderly patients, with a majority being male, and mainly manifesting as rapidly progressive episodic amnesia, depression, anxiety, behavioral abnormalities, and psychiatric symptoms.

The term "anti-Hu antibody-associated encephalitis" in the present invention is also referred to as anti-neuronal nuclear antibody type I-associated encephalitis, and anti-Hu antibody is an intracellular antibody mainly seen in small cell lung cancer, and approximately 3/4 of the patients positive for this antibody have small cell lung cancer. The lesions caused by anti-Hu antibodies mainly affect the limbic system, and can also affect the brainstem, cerebellum, spinal cord and other sites, and the clinical manifestations of patients mainly include recent memory decline, psychiatric and behavioral abnormalities and epileptic seizures, and also often affect the cerebellum, manifesting as subacute cerebellar degeneration; it can also affect peripheral nerves, manifesting as subacute sensory neuropathy or sensory/motor neuropathy.

The term "anti-CV2 antibody-associated encephalitis" in the present invention has clinical manifestations of memory loss, psychiatric and behavioral abnormalities, and epileptic seizures, and chorea-like movement abnormalities are common, and a few cases may have Parkinson-like symptoms.

The term "anti-Ma antibody-associated encephalitis" in the present invention is an autoimmune encephalitis associated with anti-intracellular antigen antibody, and anti-Ma (Ma1/Ma2/Ma3) is one of the paraneoplastic-associated neural tumor antigens, and in addition to clinical manifestations such as cognitive decline, psychiatric symptoms and epilepsy, symptoms of upper brainstem and diencephalic dysfunction may also be present, and it is mostly closely related to tumor occurrence.

The term "anti-Kelch-like protein 11 (KLHL11) antibody-associated encephalitis" in the present invention refers to a paraneoplastic rhombencephalitis associated with testicular seminoma, and a few cases may have limbic encephalitis symptoms. Vertigo, hearing loss, and tinnitus are common and unique clinical symptoms in patients with this disease.

The term "anti-Ri antibody-associated encephalitis" in the present invention is also referred to as "anti-neuronal nuclear antibody type 2-associated encephalitis", the Ri antigen is localized to neurons of the central nervous system, and anti-Ri antibodies are mainly seen in the brain, brainstem, and spinal cord, and are one of the specific markers of breast cancer or small cell lung cancer.

The term "anti-Yo antibody-associated encephalitis" in the present invention is also referred to as "Purkinje cell antigen type I antibody-associated encephalitis", and anti-Yo antibody is a polyclonal complement-binding IgG-type antibody that mainly acts on the Yo antigen in cerebellar Purkinje cells, and the Yo antigen is a cytoplasmic protein. The typical symptom of anti-Yo antibody-associated encephalitis is subacute cerebellar ataxia, manifesting as ataxia involving the trunk and limbs, vertigo, nystagmus and dysarthria, and it can also manifest as severe encephalomyelitis, and can also manifest as brainstem encephalitis, tetraplegia and disturbance of consciousness.

The term "anti-VGKC encephalitis" in the present invention refers to anti-VGKC (voltage-gated potassium channel) encephalitis, which is the most common type of neuronal surface AE, and mainly manifests as typical limbic lobe encephalitis, especially as progressive refractory epilepsy.

The term "anti-VGCC encephalitis" in the present invention refers to anti-VGCC (voltage-gated calcium channel receptor) encephalitis, which is less common in women and children, and has the prominent manifestation of affecting structures outside the limbic system, with gyral-like enhancement and cortical laminar necrosis.

The term "immune checkpoint inhibitor-associated encephalitis" in the present invention refers to autoimmune encephalitis caused after the use of immune checkpoint inhibitors (ICIs). The related clinical manifestations may include headache, fever, weakness, fatigue, confusion, disorientation and attention disorder, memory disorder, somnolence, hallucinations, epileptic seizures, and neck stiffness.

The term "biologic product-based medicament treatment regimen" in the present invention is commonly used for the treatment of patients with traditional treatment failure, glucocorticoid resistance or intolerance, relapse during glucocorticoid tapering, refractory or severe cases, and currently promising biological targeted therapies exemplarily include:
(1) B cell depletion therapy: such as, non-limitatively, anti-CD20 monoclonal antibody, anti-CD19 monoclonal antibody, B lymphocyte activating factor (BAFF) inhibitor and the like;
(2) targeting T lymphocytes: such as, non-limitatively, abatacept, signaling lymphocytic activation molecule family member 7 (SLAMF 7) monoclonal antibody, inducible co-stimulatory molecule ligand (ICOSL) inhibitor and the like;
(3) cytokine-targeting agents (IL-4, IL-5\TNF-α) and intracellular signaling pathway-targeting JAK inhibitors, and the like.

The term "about" in the present invention is used to indicate that a numerical value includes the inherent error variation of the device or method used to determine the numerical value, or the variation existing between samples being measured. Unless otherwise specified or apparent from the context, the term "about" means within 10% above or below the reported value (unless the number would exceed 100% of a possible value or fall below 0%). When used in conjunction with a numerical range or series, unless otherwise stated, the term "about" applies to the endpoints of the range or each numerical value listed in the series.

The implementations of the present invention will be described in detail below in conjunction with embodiments, but those skilled in the art will understand that the following embodiments are only used for illustrating the present invention and should not be regarded as limiting the scope of the present invention.

### Example 1 Affinity Study

The affinities of RC18 and ALPN-303 with APRIL and BAFF were compared.

The affinities of RC18 and ALPN-303 with APRIL homotrimers and Blys homotrimers were tested respectively using enzyme-linked immunosorbent assay (ELISA) method, wherein null-IgG1 was used as a blank control. The experimental results are shown in detail in Table 4.

**Table 4. April or Blys homotrimer ELISA Binding Assay**

| Sample | April (EC50, nM) | Blys (EC50, nM) |
|---|---|---|
| RC18 | 1.104 | 0.219 |
| ALPN-303 | 0.128 | 0.057 |
| null-IgG1 | N.B | N.B |

| | | |
|---|---|---|
| Note: "N.B" indicates no binding. | | |

### Conclusion

In the April or Blys homotrimeric ELISA binding assay, both RC18 and ALPN-303 achieved good binding to April and Blys.

### Example 2 Therapeutic Effect of Real-World Cases

### [Case 1]

The patient successively developed symptoms of dizziness, unstable gait, blurred vision, restricted eye movement, and involuntary head movements, and was clinically diagnosed with autoimmune encephalitis (GAD65 type) based on multiple diagnostic indicators.

The patient received neurotrophic therapy, circulatory support, and glucocorticoid pulse therapy, and was administered rituximab. Following treatment, the patient developed generalized tremors and pallor, and the infusion was immediately discontinued. Subsequently, treatment with an immunosuppressant (mycophenolate mofetil 0.75 g bid) +glucocorticoid (prednisone 60 mg qd) was administered, but the patient continued to experience dizziness and an unsteady gait. Thereafter, treatment with immunosuppressants (cyclophosphamide 0.8 g qd, mycophenolate mofetil 0.75 g bid) + glucocorticoid (prednisone 45 mg qd) was administered again, but no clinical improvement was observed. The prednisone dosage was later adjusted (30mg qd); the patient's condition remained unchanged, and the patient was unable to ambulate. Subsequently, the treatment regimen was adjusted to an immunosuppressant (mycophenolate mofetil 0.75 g bid) + glucocorticoid (prednisone 15 mg qd) + telitacicept (160 mg, once a week), and the treatment was effective, with CD19-positive lymphocytes accounting for 0.26% on peripheral blood examination, and serum immunoglobulin levels were within the normal range.

The results showed:
① For AE patients who have difficulty tolerating glucocorticoids + rituximab, telitacicept can serve as an ideal alternative therapeutic medicament, with efficacy superior to glucocorticoids + rituximab and fewer adverse reactions.
② For patients whose condition still showed no improvement after repeated treatment with glucocorticoid + rituximab and glucocorticoid + immunosuppressant, after telitacicept treatment, serum immunoglobulin returned to normal and the patient's condition was relieved.

In summary, compared with existing treatment regimens and methods, telitacicept demonstrates better safety and efficacy in the treatment of patients with autoimmune encephalitis.

### [Case 2]

The patient successively developed headache of unknown origin and low-grade fever, accompanied by limb weakness and progressive inability to ambulate. The patient additionally developed slurred speech, inappropriate vocalizations, inability to communicate, along with concurrent dysphagia, and urinary and fecal incontinence. Cellular localization demonstrated positivity for astrocyte antibodies and neuronal antibodies, with strong positivity (+++), and in conjunction with other clinical results, the diagnosis of autoimmune encephalitis was established.

The patient had previously received anti-anxiety and antidepressant therapy, neuroprotective therapy, and neurotrophic support, with no significant improvement.

Subsequently, glucocorticoid (methylprednisolone 120 mg, ivd, qd) + intravenous human immunoglobulin (15 g, ivd, qd) was administered, along with symptomatic treatments including antiepileptic therapy, anti-infective therapy, management of psychiatric symptoms, and correction of electrolyte disturbances. The patient then developed sudden status epilepticus, hypotension, tachycardia, dyspnea, unresponsiveness to verbal stimuli, and coma.

On this basis, tracheal intubation and double plasma exchange treatment were performed 3 times, and epileptic seizures still occurred. Thereafter, the glucocorticoid was tapered, and an immunosuppressant (mycophenolate mofetil 0.5 g bid) was added, with subsequent improvement. Following disease relapse, the patient returned for follow-up and underwent two sessions of immunoadsorption. The glucocorticoid was further tapered, mycophenolate mofetil (0.5 g bid) was continued, and symptomatic treatments including antiepileptic therapy and management of psychiatric symptoms were maintained.

Upon another relapse, the patient returned for follow-up and was treated with telitacicept (160 mg qw), while the glucocorticoid (methylprednisolone 8 mg qd) was further tapered, along with supportive symptomatic management.

After combination therapy with glucocorticoid and telitacicept, the patient's condition improved significantly. Psychiatric symptoms were controlled, the patient regained the ability to communicate and showed no signs of infection, muscle strength was improved, and liver and renal function were generally within normal limits.

The results showed:
① Compared with the glucocorticoid and glucocorticoid + immunosuppressant treatment regimens, the glucocorticoid + telitacicept treatment regimen demonstrated superior therapeutic effect.
② In patients with a longer disease course and recurrent relapses despite prior treatment, telitacicept exhibited favorable therapeutic potential.

### [Case 3]

The patient successively developed mental status changes, mood disturbance, and severe insomnia, accompanied by sleep talking, excessive sweating, and urinary frequency. The patient also experienced bilateral lower limb weakness, burning sensation in both feet, bilateral lower limb pain, muscle tremors, and gait instability for 2 months, with a weight loss of 15 kg. Autoimmune encephalitis antibody panel testing (six-item panel) showed serum positivity for anti-contactin-associated protein-like 2 (CASPR2) IgG antibody, while the remaining antibodies were negative. Based on the clinical findings, the patient was diagnosed with autoimmune encephalitis, Morvan syndrome subtype.

The patient was given methylprednisolone pulse therapy and gradually tapered off (200 mg-160 mg-80 mg-40 mg), and telitacicept 160 mg qw subcutaneous injection was started for 3 months, with symptomatic treatment during the period: gabapentin, anti-anxiety and antidepression. Weakness, pain, and muscle tremor in both lower limbs improved; Memory, mood, and sleep improved (MMSE 30, Moca 28, HAMA 8, HAMD 12); Hyperhidrosis resolved, and urinary frequency improved. The glucocorticoid was successfully tapered to prednisone 15 mg/d, and the heart rate decreased to 94 bpm. Tacrolimus maintenance therapy was subsequently initiated.

The results showed:
After 3 months of treatment with glucocorticoid combined with telitacicept, the patient's symptoms improved significantly, and the glucocorticoid dose was successfully tapered, demonstrating the therapeutic efficacy of telitacicept.

### [Case 4]

The patient had a subacute onset, primarily presenting with episodic limb convulsions, loss of consciousness, and speech impairment. Physical examination revealed word-finding difficulty. Brain MRI demonstrated increased meningeal enhancement in the cerebral hemispheres. Autoimmune encephalitis testing showed positive anti-NMDAR and anti-MOG antibodies. Electroencephalography was abnormal. Based on the above findings in conjunction with other clinical results, the diagnosis of autoimmune encephalitis with dual positivity for MOG and NMDAR antibodies was established.

The patient received gamma globulin (0.4 g/kg/d for 5 consecutive days). Glucocorticoid therapy was administered at 500 mg/d for 3 days, tapered to 240 mg/d for 3 days, then to 120 mg/d for 3 days, and subsequently reduced to methylprednisolone 60 mg/d orally. The dose was further tapered by 5 mg every 5 days. Due to laboratory findings indicating an increased risk of glucocorticoid-induced femoral head osteonecrosis, and in consideration of the patient's strong desire to reduce glucocorticoid exposure, telitacicept 160 mg was initiated subcutaneously once weekly. Supportive treatment included potassium supplementation, gastric mucosal protection, nutritional support, and symptomatic therapy. Antiepileptic treatment consisted of sodium valproate and levetiracetam.

At the first follow-up, no further episodic limb convulsions, loss of consciousness, or speech impairment were observed, and clinical symptoms were improved. The glucocorticoid was successfully tapered to methylprednisolone 12 mg/d and continued to be gradually reduced. Telitacicept 160 mg was administered subcutaneously once weekly, regularly administered for 12 weeks. The dosing interval was subsequently extended to 160 mg once every 10 days. Repeat testing showed that serum anti-NMDAR and anti-MOG antibodies were negative. Repeat electroencephalography was normal. Repeat blood routine examination, liver and renal function tests were within normal limits, and the serum concentration of sodium valproate was within the therapeutic range.

At the second follow-up, no further episodic limb convulsions, loss of consciousness, or speech impairment were observed, and clinical symptoms had completely resolved. Glucocorticoid therapy (methylprednisolone) was gradually tapered and discontinued. Telitacicept 160 mg was administered subcutaneously once weekly for 12 weeks, after which the dosing interval was extended to 160 mg once every 10 days. Repeat blood routine examination, liver and renal function remained within normal limits, and the serum concentration of sodium valproate remained within the therapeutic range.

The results showed:
The patient received intravenous immunoglobulin and glucocorticoid therapy in combination with telitacicept for 3 months. Significant clinical improvement was observed. Intravenous immunoglobulin and glucocorticoid therapy were successfully discontinued, and telitacicept maintenance therapy was continued. At subsequent follow-up, symptoms had completely resolved, serum antibodies converted to negative, and electroencephalography returned to normal, demonstrating the therapeutic efficacy of telitacicept.

### Example 3 Clinical Study

### Primary Objective

To evaluate the efficacy of telitacicept in the treatment or remission of autoimmune encephalitis.

### Secondary Objective

To preliminarily assess the safety of telitacicept in the treatment or remission of autoimmune encephalitis.

The above description is only of preferred embodiments, which serve as examples only and do not limit the combination of features necessary for carrying out the present invention. The headings provided are not intended to limit the various embodiments of the present invention. Terms such as "comprising", "containing" and "including" are not intended to be limiting. Furthermore, unless otherwise stated, the singular includes the plural when not modified by a numeral modifier, and "or" means "and/or". Unless defined otherwise herein, all technical and scientific terms used herein have the same meanings as commonly understood by one of ordinary skill in the art.

All publications and patents mentioned in this application are incorporated herein by reference. Various modifications and variations of the described methods and compositions of the present invention will be apparent to one of ordinary skill in the art without departing from the scope and spirit of the present invention. Although the present invention has been described by way of specific preferred implementations, it should be understood that the invention as claimed should not be unduly limited to these specific implementations. In fact, various modifications of the described modes for carrying out the present invention that are apparent to one of ordinary skill in the relevant art are intended to be within the scope of the appended claims.

## Claims

1. A method for treating or alleviating autoimmune encephalitis, the method comprising administering a therapeutically effective amount of a medicament targeting Blys and/or APRIL to a patient having the autoimmune encephalitis.

2. The method of claim 1, wherein the medicament targeting Blys and/or APRIL is a TACI-Fc fusion protein.

3. The method of claim 2, wherein the TACI-Fc fusion protein comprises:
(i) a TACI extracellular region or a fragment thereof that binds Blys and/or APRIL; and
(ii) a human immunoglobulin constant region fragment.

4. The method of claim 3, wherein the TACI extracellular region or a fragment thereof that binds Blys and/or APRIL comprises the amino acid sequence set forth in SEQ ID NO:1 or SEQ ID NO:2 or SEQ ID NO:3.

5. The method of claim 3, wherein the human immunoglobulin is IgG1, or the human immunoglobulin constant region fragment comprises the amino acid sequence of SEQ ID NO:4 or comprises an amino acid sequence having at least 90%, at least 91%, at least 92%, at least 93%, at least 94%, at least 95%, at least 96%, at least 97%, at least 98%, or at least 99% identity to SEQ ID NO:4.

6. The method of claim 5, wherein the human immunoglobulin constant region fragment comprises a modification of an amino acid at one or more positions corresponding to positions 3, 8, 14, 15, 17, 110, 111 or 173 of SEQ ID NO:4; preferably, the human immunoglobulin constant region fragment comprises amino acid modifications at 1, 2, 3, 4, 5, 6, 7, 8 or more positions as compared to SEQ ID NO:4.

7. The method of claim 6, wherein the modification is a substitution, deletion, or insertion of amino acids; preferably, the substitution comprises one or more of P3T, L8P, L14A, L15E, G17A, A110S, P111S, and A173T; preferably, the insertion is the insertion of 1, 2, 3, 4, 5, 6, 7, 8 or more amino acids at the N-terminus of the human immunoglobulin constant region fragment.

8. The method of claim 6, wherein the human immunoglobulin constant region fragment comprises the amino acid sequence of SEQ ID NO:5 or SEQ ID NO:6.

9. The method of claim 8, wherein the TACI-Fc fusion protein has an amino acid sequence with at least 75%, at least 80%, at least 85%, at least 90%, at least 95% or at least 99% identity to SEQ ID NO:7.

10. The method of claim 8 or 9, wherein the TACI-Fc fusion protein has the amino acid sequence set forth in SEQ ID NO:7 or SEQ ID NO:8 or SEQ ID NO:9.

11. The method of claim 10, wherein the TACI-Fc fusion protein is telitacicept, atacicept, or povetacicept.

12. The method of any one of claims 1 to 11, wherein the autoimmune encephalitis includes, but is not limited to, anti-cell surface antigen antibody encephalitis, anti-intracellular synaptic antigen antibody encephalitis, and anti-intracellular antigen antibody encephalitis.

13. The method of any one of claims 1 to 12, wherein the autoimmune encephalitis includes, but is not limited to, one or more of: anti-NMDAR encephalitis, Morvan syndrome, progressive encephalomyelitis with rigidity and myoclonus, anti-dipeptidyl-peptidase-like protein-6 (DPPX) antibody encephalitis, anti-dopamine 2 receptor antibody-associated basal ganglia encephalitis, anti-LGI1 antibody-associated encephalitis, anti-GABABR antibody-associated encephalitis, anti-CASPR2 antibody-associated encephalitis, anti-IgLON5 antibody-associated encephalopathy, anti-AMPAR antibody-associated encephalitis, anti-GABAAR antibody-associated encephalitis, anti-mGluR5 antibody-associated encephalitis, anti-neurexin-3α antibody-associated encephalitis, anti-glutamic acid decarboxylase (GAD) antibody encephalitis, anti-AK5 antibody-associated encephalitis, anti-Hu antibody-associated encephalitis, anti-CV2 antibody-associated encephalitis, anti-Ma antibody-associated encephalitis, anti-Kelch-like protein 11 antibody-associated encephalitis, anti-Ri antibody-associated encephalitis, anti-Yo antibody-associated encephalitis, anti-VGKC encephalitis, anti-VGCC encephalitis, and immune checkpoint inhibitor-associated encephalitis; preferably, the anti-glutamic acid decarboxylase (GAD) antibody encephalitis is selected from anti-glutamic acid decarboxylase (GAD) 65 antibody encephalitis; preferably, the autoimmune encephalitis comprises anti-NMDAR encephalitis, Morvan syndrome, and anti-glutamic acid decarboxylase (GAD) 65 antibody encephalitis.

14. The method of any one of claims 1 to 13, wherein the autoimmune encephalitis patient is in a new-onset disease treatment stage or a relapsing disease treatment stage; preferably, the patient is an adult patient or a pediatric patient; further preferably, the patient is an antibody-positive autoimmune encephalitis patient or an antibody-negative autoimmune encephalitis patient; further preferably, the antibody-positive autoimmune encephalitis patient is a patient with autoimmune encephalitis positive for one or more antibodies.

15. The method of any one of claims 1 to 14, wherein the autoimmune encephalitis patient concurrently suffers from one or more other autoimmune diseases; preferably, the autoimmune encephalitis patient concurrently suffers from one or more other tumor diseases.

16. The method of any one of claims 1 to 15, wherein the patient has previously received an autoimmune encephalitis treatment regimen or has not previously received an autoimmune encephalitis treatment regimen.

17. The method of any one of claims 1 to 16, wherein the autoimmune encephalitis treatment regimen comprises, but is not limited to, one or more of a glucocorticoid treatment regimen, an immunosuppressant treatment regimen, a biologic product treatment regimen, and plasma exchange; preferably, the glucocorticoid is selected from prednisone and/or methylprednisolone; preferably, the immunosuppressant is selected from cyclophosphamide and/or mycophenolate mofetil; preferably, the biologic product is selected from one or more of rituximab, human immunoglobulin, and gamma globulin.

18. The method of any one of claims 1-17, wherein the method comprises administering to the patient having the autoimmune encephalitis a therapeutically effective amount of a medicament targeting Blys and/or APRIL in combination with one or more of a glucocorticoid, an immunosuppressant, biologic product, and plasma exchange; preferably, the immunosuppressant is mycophenolate mofetil; preferably, the glucocorticoid is prednisone or methylprednisolone; preferably, the biologic product is selected from one or more of rituximab, human immunoglobulin, and gamma globulin; further preferably, administering a therapeutically effective amount of a medicament targeting Blys and/or APRIL in combination with a glucocorticoid, wherein the medicament targeting Blys and/or APRIL is administered once a week and the glucocorticoid is administered once a day; further preferably, administering a therapeutically effective amount of a medicament targeting Blys and/or APRIL in combination with a glucocorticoid and an immunosuppressant, wherein the medicament targeting Blys and/or APRIL is administered once a week, and the glucocorticoid is administered once a day, and the immunosuppressant is administered twice a day.

19. The method of any one of claims 1 to 18, wherein a single dose of the medicament targeting Blys and/or APRIL is about 0.1 to 10 mg/kg; preferably, a single dose of the medicament targeting Blys and/or APRIL is 40-240 mg, more preferably 40 mg, 60 mg, 80 mg, 100 mg, 120 mg, 140 mg, 160 mg, or 240 mg.

20. The method of any one of claims 1 to 19, wherein a dosing frequency of the medicament targeting Blys and/or APRIL is as-needed dosing; preferably, the medicament targeting Blys and/or APRIL is administered 1-5 times per month, and/or 1-10 times per two-month interval, and/or 1-15 times per three-month interval; further preferably, a dosing frequency of the medicament targeting Blys and/or APRIL is once a week or once every 10 days; further preferably, an administration mode of the medicament targeting Blys and/or APRIL is subcutaneous, intramuscular, or intravenous administration, and an administration site is the thigh, the abdomen, or the upper arm.
